(19) Europäisches Patentamt
European Patent Office
Office européen des brevets

(11) Veröffentlichungsnummer: **0 034 285**
**B1**

(12) **EUROPÄISCHE PATENTSCHRIFT**

(45) Veröffentlichungstag der Patentschrift: **06.11.85**

(51) Int. Cl.[4]: **C 07 C 109/04**

(21) Anmeldenummer: **81100664.2**

(22) Anmeldetag: **30.01.81**

# (54) Verfahren zur Herstellung von 1-Alkyl-1-Phenylhydrazinen.

(30) Priorität: **06.02.80 DE 3004206**

(43) Veröffentlichungstag der Anmeldung: **26.08.81 Patentblatt 81/34**

(45) Bekanntmachung des Hinweises auf die Patenterteilung: **06.11.85 Patentblatt 85/45**

(84) Benannte Vertragsstaaten: **CH DE FR GB LI**

(56) Entgegenhaltungen:
**DE - C - 890 648**

**JOURNAL OF THE AMERICAN CHMEICAL SOCIETY, Band 99, Nr. 14, 6 Juli 1977, Pennsylvania, P.A.S. SMITH et al. "Hydrazines as ambident nucleophiles: the site of benzylation of N-Benzyl-N-phenylhydrazines" "Beilsteins Handbuch der Organischen Chemie", 4. Auflage, Band XV, 3. Ergänzungswerk, 1974, SPRINGER-VERLAG, Berlin HOUBEN-WEYL "Methoden der organischen Chemie", 4. Auflage, Band X/2, Teil 2, 1967, GEROG THIEME VERLAG, Stuttgart**

(73) Patentinhaber: **HOECHST AKTIENGESELLSCHAFT, Postfach 80 03 20, D-6230 Frankfurt am Main 80 (DE)**

(72) Erfinder: **Lerch, Ulrich, Dr., Schwalbenweg 19, D-6238 Hofheim am Taunus (DE)**
Erfinder: **König, Johann, Im Kirschgarten 4, D-6239 Eppstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

α-alkylierte Phenylhydrazine sind wichtige Vor- und Zwischenprodukte auf vielen Gebieten; insbesondere stellen sie wertvolle Vorprodukte für die Herstellung von Pharmazeutika dar, z.B. dienen sie als Ausgangsmaterialien für die Herstellung von 1-alkylierten Indolen, 1-Alkyl-1-phenylhydrazonen oder von 1-Alkyl-1-phenylhydraziden. Für die Synthese von α-alkylierten Phenylhydrazinen existiert eine Reihe von Methoden (vgl. Houben-Weyl Band 10/2, Seite 396ff, G. Thieme Verlag, Stuttgart 1967). Eine häufig verwendete Darstellungsmethode beruht auf der Nitrosierung von N-Alkylanilinen und nachfolgender Reduktion des N-Nitroso-N-alkylanilins [vgl. Org. Synth. Coll. Vo. 2, S. 418 (1943), J. Amer. Chem. Soc. 74, 3693 (1952), ibid 77, 790 (1955), Act Chem. Scand 1971, 2337, US Pat. 3 102 887, DOS 2 758 027]. Einige der bekannten Methoden zur Herstellung von 1-Alkyl-1-phenylhydrazinen erfordern die Verwendung von Schutzgruppen und damit eine umständliche Synthese über drei Stufen [vgl. Ber. 18, 1744 (1885), Compt. rend. 209, 994 (1939), Synthesis 1976, 795] mit einem mässigen oder unbefriedigenden quantitativen Resultat.

Die direkte Alkylierung von Phenylhydrazin wurde bisher in flüssigem Ammoniak oder in unpolaren Lösungsmitteln wie Benzol oder Xylol beschrieben. Für die Umsetzung in Benzol und Xylol wurden als Basen Natriummetall und Natriumamid verwendet.

Bei der Natriummetall-Methode wurde das Natriumsalz des Phenylhydrazins in Substanz isoliert (vgl. Ber. 30, 2810, Ber. 38, 866, Ann. 252, 266) und als solches in einem unpolaren Lösungsmittel wie Benzol mit dem Alkylierungsmittel zur Reaktion gebracht. Abgesehen von der Gefährlichkeit dieser Methode [vgl. J. chem. Soc. 71, 462 (1897)] ergab dieses Vorgehen unreine Produkte in schlechter Ausbeute.

Auch die Verwendung von Natriumamid in Benzol (vgl. J. chem. Soc. 1943, 67) ergibt schwer trennbare Gemische von Alkylanilin, Alkylphenylhydrazin und unverändertem Phenylhydrazin.

Ein nach diesem Verfahren mit Methyljodid erhaltenes Produkt (etwa 70% Gesamtausbeute), enthielt gemäss einer gaschromatographischen Analyse mehr als 50% N-Methylanilin.

Die Umsetzung von Phenylhydrazin mit Natrium- oder Kaliumamid in flüssigem Ammoniak und nachfolgender Zugabe von Alkylierungsmittel stellte bisher die beste Methode zur selektiven α-Alkylierung von Phenylhydrazin dar [vgl. J. Org. Chem. 6, 416 (1941)]. Dieses Verfahren hat jedoch den Nachteil, dass man bei tiefen Temperaturen (unter −35°) und mit grossen Mengen flüssigem Ammoniak arbeiten muss. Dies erfordert zusätzlichen technischen Aufwand und Energiebedarf und verursacht Umwelt-Probleme bei der Beseitigung des Ammoniaks.

Es wurde ein Verfahren zur Herstellung von 1-Alkyl-1-Phenylhydrazin der Formel I gefunden

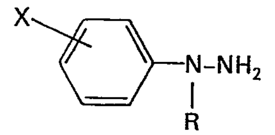

I

worin bedeuten

X Wasserstoff oder einen reaktionsinerten Substituenten, und

R Alkyl oder Alkenyl mit 1 bis 15 C-Atomen, in dem eine $CH_2$ bzw. CH-Gruppe durch ein Sauerstoffatom ersetzt sein kann, wobei die Sauerstofffunktion durch mindestens zwei Kohlenstoffatome vom Stickstoff des Phenylhydrazins getrennt ist, Cycloalkyl oder Cycloalkenyl mit 3 bis 7 C-Atomen, Phenylalkyl mit 1 bis 6 C-Atomen, oder Cycloalkylalkyl mit 1–6 Alkyl- und 3 bis 7 Cycloalkyl-C-Atomen durch Umsetzung eines Phenylhydrazins der Formel II

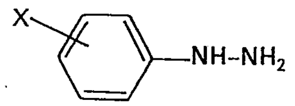

II

mit einer starken Base und mit einem Alkylierungsmittel der Formel III

R–Y III

worin

Y Chlor, Brom oder Jod, Alkyl- oder Arylsulfonyloxy, oder, sofern R niedrig Alkyl bedeutet, $-O-SO_2OR$ bedeutet, in einer organischen Flüssigkeit, das dadurch gekennzeichnet ist, dass man mit $NaNH_2$ als starker Base in einem aprotischen Lösungsmittel ausgewählt aus der Gruppe Tetrahydrofuran, 1,2-Dimethoxyethan, Acetonitril und/oder Dioxan deprotoniert.

Als Ausgangsmaterial für dieses Verfahren dienen in erster Linie solche Phenylhydrazine der Formel II, in denen X Wasserstoff, Halogen, Alkyl mit 1–6 C-Atomen oder Alkoxy mit 1–6 C-Atomen ist.

Als Verbindungen der Formel III kommen vor allem solche in Frage, in denen

R einen gradkettigen oder verzweigten, gesättigten oder ungesättigten aliphatischen Rest mit 1–15 C-Atomen, in dem eine $CH_2$ bzw. CH-Gruppe durch ein Sauerstoffatom ersetzt sein kann, wobei die Sauerstoffunktion durch mindestens zwei Kohlenstoffatome von Y getrennt ist, oder einen cycloaliphatischen Rest mit 3–7 Kohlenstoffatomen oder einen Alkylrest mit 1–6 Kohlenstoffatomen, der seinerseits durch einen ggf. substituierten Phenylrest oder einen Cycloalkylrest mit 3–7 Kohlenstoffatomen substituiert sein kann, bedeutet.

Überraschenderweise verlaufen die Alkylierungen von Phenylhydrazinen nach dem erfindungsgemässen Verfahren selektiv am α-Stickstoffatom und liefern 1-Alkyl-1-phenylhydrazine in hohen Ausbeuten und Reinheitsgraden.

Dies gilt ganz besonders, wenn ein Lösungsmittel benutzt wird, in dem das entsprechende Metall-phenylhydrazid ganz oder teilweise löslich

ist. Solche Lösungsmittel sind in erster Linie polare, aprotische Lösungsmittel, wie Dimethylformamid, Dimethylacetamid, N-Methylpyrrolidon, Solvolan, Hexamethylphosphortriamid, Acetonitril oder Diglycoldimethyläther. Besonders bevorzugt sind Tetrahydrofuran, 1,2-Dimethoxyäthan und Dioxan.

Als Basen zur Herstellung des Metall-phenylhydrazids sind alle jene geeignet, deren Basenstärke ausreicht, um das jeweils verwendete Phenylhydrazin unter den Reaktionsbedingungen im Gleichgewicht oder vollständig zu deprotonieren. Es kommen hierfür insbesondere metallorganische Verbindungen in Frage, wie Methyllithium, Phenyllithium, Butyllithium, Triphenylmethylnatrium, Natriumhexamethyldisilazan, Natriummethylsulfinylmethid, Methylmagnesiumjodid oder Phenylmagnesiumbromid; Metallalkoholate wie Natriumäthylat oder Kaliumtert.-butylat; Metallhydride wie Natirumhydrid, Kaliumhydrid, Lithiumhydrid oder Aluminiumhydrid; Metallamide, die sich von organischen Aminen oder vom Ammoniak ableiten, z.B. Lithiumdiisopropylamid, Natriumamid, Kaliumamid oder Lithiumamid.

Die Alkylierungsreaktion nach dem erfindungsgemässen Verfahren wird zweckmässigerweise unter einer Inertgas-Atmosphäre durchgeführt, vorzugsweise unter Stickstoff oder Argon, da das Metallphenylhydrazid sauerstoffempfindlich ist.

Bei einer bevorzugten Ausführungsweise des Verfahrens wird das Lösungsmittel und die Base unter Schutzgas vorgelegt und das Phenylhydrazin zu dieser Mischung zugegeben. Es ist jedoch auch möglich, das Phenylhydrazin in dem gewählten Lösungsmittel vorzulegen und anschliessend die Base zuzufügen.

Die Reaktionstemperatur für die Deprotonierung des Phenylhydrazins ist von der Natur der verwendeten Base abhängig. Wegen der Thermolabilität der N-N-Bindung von Phenylhydrazinen [vgl. J. Chem. Soc. 99, 408 (1911)] arbeitet man zweckmässigerweise unterhalb von 50°C, besonders bevorzugt sind Temperaturen unterhalb von 20°. Z.B. kann man bei der Verwendung von Natriumamid zwischen -20 und +30°, vorteilhafterweise zwischen 0 und 10° arbeiten. Mit sehr starken metallorganischen Basen wie z.B. Butyllithium oder Lithiumdiisopropylamid können Temperaturen bis -80° für die Deprotonierungsreaktion empfehlenswert sein.

Sofern man ein Alkaliamid, wie Natriumamid verwendet, ist es vorteilhaft, vor der Zugabe des Alkylierungsmittels den entstandenen Ammoniak zu entfernen. Dies kann in der Weise geschehen, dass man längere Zeit, z.B. ein bis zwei Stunden, Inertgas durch die Reaktionslösung leitet.

Die Alkylierung des gebildeten Metallphenylhydrazids erfolgt bei Temperaturen zwischen -30 und +100°. Bevorzugt sind auch hier tiefe Temperaturen. Die Reaktionsgeschwindigkeit und Temperatur ist naturgemäss von der Art des verwendeten Alkylierungsmittels abhängig. Mit Alkyljodiden arbeitet man z.B. zweckmässig bei Temperaturen zwischen -10 und +30°, vorzugsweise zwischen 0 und 20°; mit Alkylchloriden zwischen 10 und 60°C, vorzugsweise zwischen 30 und 50°.

Bei dieser Umsetzung empfiehlt es sich, zu der Lösung des Metallphenylhydrazids bei der gewählten Temperatur das Alkylierungsmittel, gegebenenfalls gelöst in einem der oben genannten aprotischen polaren Lösungsmittel, zuzutropfen und nach Beendigung der Zugabe bis zur Beendigung der Reaktion weiter zu rühren.

Das Alkylierungsmittel wird zweckmässig in geringem molarem Überschuss angewandt. Pro Mol Phenylhydrazin werden demgemäss etwa 1,0 bis 1,3 Mol, vorzugsweise 1,0 bis 1,1 Mol Alkylierungsmittel verwendet. Der Endpunkt der Alkylierungsreaktion ist häufig dadurch erkennbar, dass sich die Farbe der Reaktionslösung aufhellt.

Zur Aufarbeitung kann man die Reaktionsmischung zwischen Wasser und einem mit Wasser nicht mischbaren organischen Lösungsmittel verteilen. Die wässrige Phase wird zweckmässigerweise mehrfach mit dem Lösungsmittel extrahiert und kann danach verworfen werden. Die vereinigten organischen Phasen können mit Wasser oder einer wässrigen Salzlösung (z.B. gesättigter Kochsalzlösung) gewaschen, getrocknet und im Vakuum eingedampft werden.

In vielen Fällen ist es vorteilhaft, das bei der Alkylierungsreaktion verwendete Lösungsmittel abzudampfen und anschliessend den Rückstand zwischen Wasser und einem mit Wasser nicht mischbaren organischen Lösungsmittel in der oben angegebenen Art und Weise zu verteilen.

Das erhaltene Rohprodukt ist gewöhnlich für alle weiteren Operationen genügend rein und kann ohne zusätzliche Reinigung direkt weiterverwendet werden. Besonders bei sehr hochsiedenden Verbindungen kann die Verwendung des Rohproduktes von Vorteil sein.

Das Rohprodukt kann nach den üblichen Reinigungsverfahren weiter gereinigt werden, z.B. mit chromatographischen Methoden, durch Kristallisation von mit Hilfe geeigneter Säuren erhaltenen Salzen oder durch Destillation.

Als chromatographische Reinigungsmethoden kommen in Betracht die Säulenchromatographie an geeigneten Trägern wie Kieselgel oder Aluminiumoxyd oder die präparative Dünnschichtchromatographie.

Eine Möglichkeit für die Reinigung ergibt sich aus der Tatsache, dass die 1-Alkyl-1-phenylhydrazine Basen sind, die mit organischen oder anorganischen Säuren Salze bilden. Sofern diese Salze kristallin sind, kann man die Produkte reinigen, indem man die Salze umkristallisiert und anschliessend aus ihnen mit einer starken Base, z.B. mit Natronlauge, die Alkylphenylhydrazine wieder in Freiheit setzt.

Bevorzugt als Reinigungsmethode ist die Destillation, die wegen der thermolabilen N-N-Bindung in den Produkten zweckmässig bei möglichst tiefen Temperaturen im Hochvakuum durchgeführt wird. Bei hochsiedenden Produkten kann dabei die Verwendung von Dünnschichtverdampfern vorteilhaft sein.

Die Verfahrensprodukte dienen als Ausgangsstoffe für chemische Synthesen, insbesondere bei der Herstellung von Pharmaka.

Die Erfindung wird durch die nachfolgenden Beispiele näher erläutert.

Beispiel 1

1-Methyl-1-phenylhydrazin

In 2,3 l Tetrahydrofuran (Merck, maximal 0,1% $H_2O$, peroxydfrei) werden unter Stickstoff bei Raumtemperatur 130 g (3,15 Mol) 95%iges Natriumamid (Degussa) eingerührt. Unter Eiskühlung tropft man in etwa 80 Minuten bei 5 bis 8° Innentemperatur 324 g (3 Mol) reines Phenylhydrazin (Riedel 99%) zu. Die Kühlung wird unterbrochen und durch die gelbe Lösung 90 Minuten lang ein mässiger Strom von Stickstoff geleitet, um das entstandene Ammoniak aus der Lösung zu entfernen. Die Innentemperatur steigt auf etwa 15°. Danach werden unter Kühlung in etwa 2,5 Stunden bei 10 bis 14° Innentemperatur 455 g (3,2 Mol) Methyljodid zugetropft, wobei gegen Ende der Reaktion Natriumjodid ausfällt und sich die Reaktionslösung fast entfärbt. Man rührt 90 Minuten unter Eiskühlung nach, lässt innerhalb von 10 Minuten 600 ml Wasser zulaufen und destilliert das Tetrahydrofuran im Wasserstrahlvakuum bei 50° Badtemperatur am Rotavapor ab. Man verdünnt den Rückstand mit weiteren 600 ml Wasser und extrahiert das abgeschiedene Öl mit 1 l und danach 2mal mit 200 ml Diisopropyläther. Die vereinigten organischen Extrakte werden 1mal mit 200 ml und 4mal mit 100 ml Wasser gewaschen, mit 40 g wasserfreiem Natriumsulfat getrocknet und im Vakuum bei etwa 40° Badtemperatur eingedampft. Das rötlich gefärbte Öl wird destilliert. $Sdp._{0,2}$ 56–57°. Ausbeute 318 g (86%) α-Methylphenylhydrazin. Das Produkt besitzt nach gaschromatographischer Analyse (25 m WCOT-Kappilarsäule belegt mit Phenylmethylsilicon) einen hohen Reinheitsgrad (etwa 98%). Hauptverunreinigung ist N-Methylanilin (etwa 1,5%).

Produkte vergleichbarer Reinheit erhält man bei Verwendung von Dimethoxyäthan (86% Ausbeute) und Dioxan (78% Ausbeute) als Lösungsmittel.

1-Methyl-1-phenylhydrazin in 83% Ausbeute wurde ähnlich dem Beispiel 1 erhalten, wenn für die Deprotonierung des Phenylhydrazins anstelle von Natriumamid Butyllithium bei -40° verwendet wurde.

Beispiel 2

1-Äthyl-1-phenylhydrazin

Die Reaktion wurde analog Beispiel 1 mit Äthyljodid als Alkylierungsmittel durchgeführt.

$Sdp._{0,3}$ 63–64° Ausbeute 91% Reinheit (GC) etwa 97%.

Beispiel 3

1-Propyl-1-phenylhydrazin

Die Reaktion wurde analog Beispiel 1 mit Propyljodid als Alkylierungsmittel durchgeführt.

$Sdp._{0,3}$ 68–70° Ausbeute 93% Reinheit (GC) etwa 97%.

Beispiel 4

1-Isopropyl-1-phenylhydrazin

Die Reaktion wurde analog Beispiel 1 mit Isopropyljodid als Alkylierungsmittel durchgeführt.

$Sdp._{0,3}$ 66–67° Ausbeute 87% Reinheit (GC) etwa 99%.

Beispiel 5

1-n-Butyl-1-phenylhydrazin

Die Reaktion wurde wie im Beispiel 1 mit n-Butylchlorid als Alkylierungsmittel durchgeführt. Wegen der geringeren Reaktivität des Butylchlorids erfolgte die Alkylierung des Natrium-Phenylhydrazins statt bei 10–14° bei 40–45° (5 Std).

$Sdp_{0,3}$ 80–82° Ausbeute 88% Reinheit (GC) etwa 98%.

Beispiel 6

1-n-Octyl-1-phenylhydrazin

Die Reaktion wurde wie im Beispiel 1 beschrieben, mit n-Octylbromid als Alkylierungsmittel durchgeführt. Die Alkylierung des Natriumphenylhydrazins erfolgte jedoch wegen der etwas geringeren Reaktionsfähigkeit des Octylbromids bei 20–25° (5 Std).

$Sdp._{0,4}$ 137–138° Ausbeute 88%.

Beispiel 7

1-Benzyl-1-phenylhydrazin

Die Reaktion wurde analog Beispiel 1 mit Benzylchlorid als Alkylierungsmittel durchgeführt.

$Sdp._{0,4}$ 113–115° Ausbeute 85%.

Beispiel 8

1-Allyl-1-phenylhydrazin

Die Reaktion wurde analog Beispiel 1 mit Allylbromid als Alkylierungsmittel durchgeführt.

$Sdp._{0,4}$ 70–71° Ausbeute 90%.

Beispiel 9

1-Methyl-1-(4-methylphenyl)-hydrazin

Die Reaktion wurde analog Beispiel 1 mit 4-Methylphenylhydrazin durchgeführt. Zur Erzielung einer guten Ausbeute ist es zweckmässig, nach der Zugabe des in THF gelösten Hydrazins zur Natriumamidsuspension 1 Stunde bei 20° nachzurühren.

$Sdp_{0,1}$ 67–68° Ausbeute 82%.

Beispiel 10

1-Methyl-1-(4-chlorphenyl)-hydrazin

Die Reaktion wurde analog Beispiel 9 mit 4-Chlorphenylhydrazin durchgeführt.

$Sdp._{0,2}$ 96–97° Ausbeute 75%.

Beispiel 11

1-(4-Methoxybut-1-yl)-1-phenylhydrazin

Die Reaktion wurde analog Beispiel 1 mit 4-Methoxybutylbromid durchgeführt. Das Produkt lässt sich nur unter teilweiser Zersetzung destillieren, ist aber für die Weiterverarbeitung genügend rein. Rohausbeute 83%.

Hydrazon mit 4-Nitrobenzaldehyd: Fp. 69–71°.

**Patentanspruch**

Verfahren zur Herstellung eines 1-Alkyl-1-phenylhydrazins der Formel I

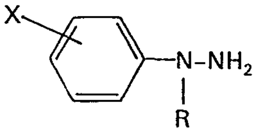

I

worin bedeuten
X Wasserstoff oder einen reaktionsinerten Substituenten, und
R Alkyl oder Alkenyl mit 1 bis 15 C-Atomen, in dem eine $CH_2$ bzw. CH-Gruppe durch ein Sauerstoffatom ersetzt sein kann, wobei die Sauerstofffunktion durch mindestens zwei Kohlenstoffatome vom Stickstoffatom des Phenylhydrazins getrennt ist, Cycloalkyl oder Cycloalkenyl mit 3 bis 7 C-Atomen Phenylalkyl mit 1 bis 6 alkyl-C-Atomen, oder Cycloalkylalkyl mit 1-6 Alkyl- und 3-7 Cycloalkyl-C-Atomen durch Umsetzung eines Phenylhydrazins der Formel II

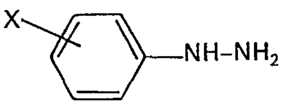

II

mit einer starken Base und mit einem Alkylierungsmittel der Formel III

R-Y III,

worin
Y Chlor, Brom oder Jod, Alkyl- oder Arylsulfonyloxy, oder, sofern R niedrig Alkyl bedeutet, $-O-SO_2-OR$ bedeutet, in einer organischen Flüssigkeit dadurch gekennzeichnet, dass man mit $NaNH_2$ als starker Base in einem aprotischen Lösungsmittel ausgewählt aus der Gruppe Tetrahydrofuran, 1,2-Dimethoxyethan, Acetonitril und/oder Dioxan deprotoniert.

**Claim**

A process for the preparation of a 1-alkyl-1-phenyl-hydrazine of the formula I

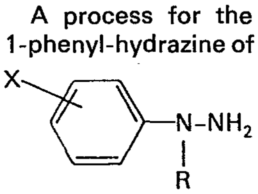

I

in which
X is hydrogen or a non-reactive substituent, and
R is alkyl or alkenyl having from 1 to 15 carbon atoms, where a $CH_2$ or CH group can be replaced by an oxygen atom, the oxygen function being separated from the nitrogen of the phenylhydrazine by at least two carbon atoms, cycloalkyl or cycloalkenyl having from 3 to 7 carbon atoms, phenylalkyl having from 1 to 6 carbon atoms, or cycloalkylalkyl having from 1 to 6 alkyl and from 3 to 7 cycloalkyl carbon atoms, by reacting a phenylhydrazine of the formula II

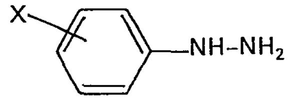

II

with a strong base with an alkylating agent of the formula III

R-Y III

in which Y is chlorine, bromine or iodine, alkyl- or arylsulfonyloxy, or, when R is lower alkyl, $-O-SO_2-OR$, in an organic liquid characterized in that one deprotonates with $NaNH_2$ as strong base in an aprotic solvent selected from the group consisting of tetrahydrofurane, 1,2-dimethoxyethane, acetonitrile and/or dioxane.

**Revendication**

Procédé de préparation d'une 1-alcoyl-1-phénylhydrazine de formule I

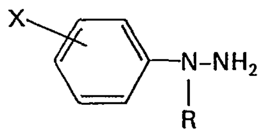

I

dans laquelle
X représente l'hydrogène ou un substituant ne réagissant pas et
R représente un groupe alcoyle ou alcényle ayant de 1 à 15 atomes de carbone, dans lequel un groupe $CH_2$ ou CH peut être remplacé par un atome d'oxygène, la fonction oxygène étant alors séparée par au moins deux atomes de carbone de l'atome d'azote de la phénylhydrazine, un groupe cycloalcoyle ou cycloalcényle ayant de 3 à 7 atomes de carbone, un groupe phénylalcoyle ayant de 1 à 6 atomes de carbone dans la partie alcoyle ou un groupe cycloalcoylalcoyle ayant de 1 à 6 atomes de carbone dans la partie alcoyle et de 3 à 7 atomes de carbone dans la partie cycloalcoyle, par réaction d'une phénylhydrazine de formule II

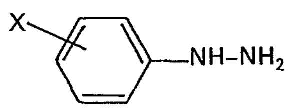

II

avec une base forte et avec un agent d'alcoylation de formule III

R-Y III

dans laquelle
Y représente le chlore, le brome ou l'iode, un groupe alcoyl ou aryl-sulfonyloxy, ou, dans la mesure où R représente un groupe alcoyle inférieur, le groupe $-O-SO_2-OR$, dans un liquide organique, ledit procédé étant caractérisé en ce qu'on déprotonise avec $NaNH_2$ comme base forte, dans un solvant aprotique choisi parmi le tétrahydrofuranne, le 1,2-diméthoxyéthane, l'acétonitrile et/ou le dioxane.